# EUROPÄISCHE PATENTSCHRIFT

(11) **EP 0 643 070 B1**
(45) Veröffentlichungstag und Bekanntmachung des Hinweises auf die Patenterteilung: **21.07.1999**
(21) Anmeldenummer: 94114188.9
(22) Anmeldetag: 09.09.1994
(51) Int. Cl.: C07J 1/00, C07J 11/00, C07J 71/00, A61K 31/565

(54) **"Steroidzwischenprodukte und Verfahren zu ihrer Herstellung"**
Steroid intermediates and process for their preparation
Produits intermédiaires stéroides et procédé pour leur préparation

(30) Priorität: 10.09.1993 DE 4330727
(43) Veröffentlichungstag der Anmeldung: 15.03.1995
(73) Patentinhaber: Jenapharm GmbH & Co. KG, 07745 Jena (DE)
(72) Erfinder: Ring, Sven, D-07749 Jena (DE); Teichmüller, Gerhard, D-07743 Jena (DE); Weber, Gisela, D-07745 Jena (DE); Prof. Schwarz, Sigfrid, D-07743 Jena (DE); Erhart, Bernd, D-07768 Kahla (DE); Dr. Undeutsch, Bernd, D-07743 Jena (DE); Dr. Räthe, Harald, D-07747 Jena (DE); Dr. Möllmann, Peter, D-07749 Jena (DE); Pfeiffer, Carmen, D-99510 Grossromstedt (DE); Dr. Palme, Hans-Joachim, D-07747 Jena (DE)

(56) Entgegenhaltungen:
- WO-A-90/11290
- FR-A- 2 209 577
- GB-A- 1 128 044
- RECUEIL DES TRAVAUX CHIMIQUES DES PAYS-BAS, Bd.107, Nr.4, April 1988 Seiten 331 - 334 M. J. VAN DEN HEUVEL ET AL 'A partial synthesis of 13-ethyl-11-methylene-18,19-d inor-17alpha-pregn-4-en-20-yn-17-ol (desogestrel) based upon intramolecular oxidation of an 11beta-hydroxy-19-norsteroid to the 18->11beta-lactone.'

## Beschreibung

Es werden neue Steroidzwischenprodukte der allgemeinen Formel Ia 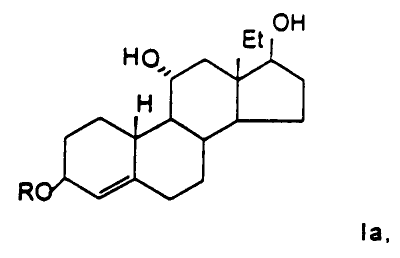 in der R ein Wasserstoffatom oder eine Methylgruppe darstellen, beschrieben.

### Es werden weiterhin Steroidzwischenprodukte der Formeln Ib bis Id

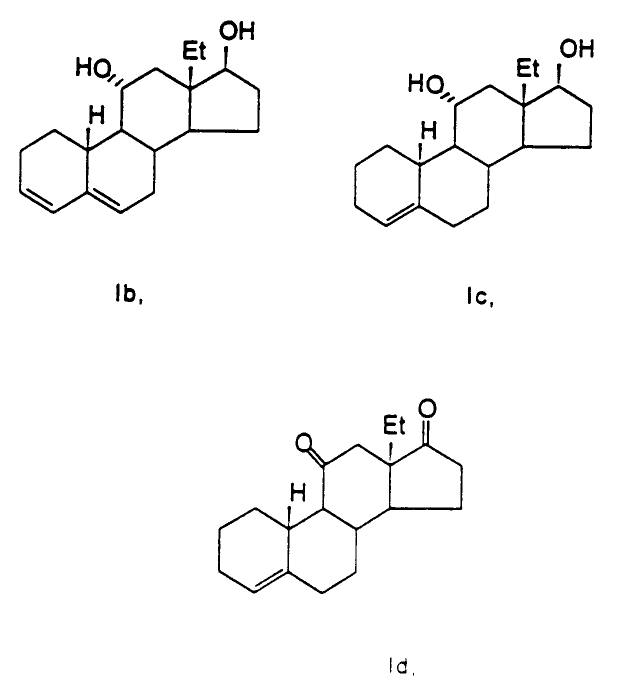

beschrieben

Ferner werden Steroidzwischenprodukte der Formeln Ie und If 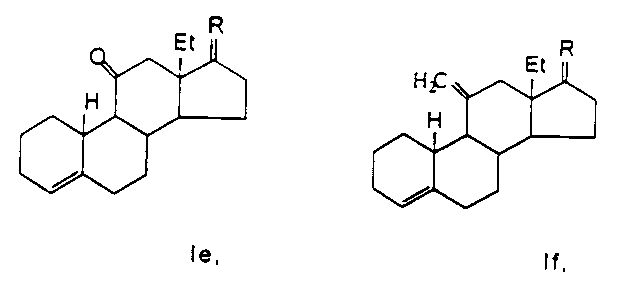 mit R= beschrieben.

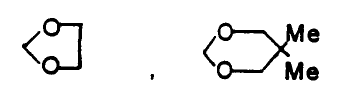

Es werden auch die Herstellungsverfahren der erfindungsgemäßen Steroidzwischenprodukte Ia bis If beschrieben.

Die erfindungsgemäß zugänglichen Steroidzwischenprodukte der Formeln Ia bis If eignen sich zur Synthese von 13-Ethyl-11-methylen-18,19-bisnor-17α-pregn-4-en-20-in-17-ol, einem Wirkstoff, der unter dem Namen Desogestrel bekannt ist. Desogstrel ist ein Gestagen, das in der hormonellen Kontrazeption eingesetzt wird (Drugs of Today, XVIII (1982), 361) und (Arzneimittel-Fortschritte 1972 bis 1985, A. Kleemann, E. Lindner und J. Engel (Hrsg.), VCH Verlagsgesellschaft D-6940 Weinheim, S. 790, 804).

Aufgabe der Erfindung ist das Auffinden neuer Steroidzwischenprodukte die, verglichen mit der bisher bekannten Synthese des Wirkstoffes Desogestrel, eine wesentlich vorteilhaftere Herstellung des Wirkstoffes gestatten.

Weiterhin ist die Aufgabe der Erfindung, effektive und umweltschonende Verfahren zur Herstellung der erfindungsgemäßen Verbindungen zu entwickeln.

Desogestrel ist nach DE 23 61 120 und DE 25 38 862 aus 3,3,17,17-Bis-ethylendioxy-estr-5-en-11β-ol zugänglich. Hierbei wird das Ausgangsmaterial durch Reaktion mit Acylhypoiodit, das aus einem Acylat eines Schwermetalls, wie Silber, Quecksilber oder Blei und elementarem Jod generiert wird , in ein 11,17-Lacton überführt, das anschließend mit einer metallorganischen Verbindung methyliert wird, wobei die 13-Acetyl-11β-hydroxy-Verbindung entsteht, die man in das 18a-Homo-11β-hydroxy-Derivat umwandelt. Im weiteren Verlauf der Synthese wird die 3-Oxo-Gruppe reduktiv aus dem Molekül entfernt, indem zunächst das 3-Thioacetal gebildet und dieses dann mit Alkalimetall in flüssigem Ammoniak gespalten wird. Die Einführung der 11-Methylen-Gruppe aus der entsprechenden 11-Oxo-Verbindung erfolgt auf dem Wege der WITTIG-Olefinierung.

Das bisher bekannt gewordene Verfahren zur Synthese von Desogestrel ist mit einer Reihe von Nachteilen behaftet.
So ist zur Bildung des 11,17-Lactons erforderlich, 3,3,17,17-Bis-ethylendioxy-estr-5-en-11β-ol mit 1,5 bis 3 Moläquivalent Jod und 2,25 bis 15 Moläquivalent Bleitetraacetat zur Reaktion zu bringen und das Reaktionsprodukt chromatographisch zu reinigen, um gute Ausbeuten zu erzielen.
Das bedeutet, daß in dieser Reaktion mit stark toxischen und umweltgefährdenden Hilfsstoffen, wie Jod und Blei- oder Quecksilberverbindungen gearbeitet werden muß.

Die reduktive Eliminierung der 3-Oxo-Gruppe aus dem Steroidmolekül über das 3-Thioacetal setzt voraus, daß zunächst das 3-Oxo-Steroid mit Ethandithiol in das Thioacetal überführt wird. Ethandithiol gehört zur Verbindungsklasse der Mercaptane, die sich durch einen ekelerregenden Geruch auszeichnen, der selbst dann noch wahrnehmbar ist, wenn das Mercaptan nur noch im ppm-Bereich vorhanden ist.

Die Einführung der 11-Methylengruppe in das Zielmolekül erfolgt durch Olefinierung eines 3-Desoxo-11-oxo-Steroids mit Methylen-triphenylphosphoran in hohem Überschuß (insgesamt 6 Moläquivalent) über einen längeren Zeitraum (22 Stunden). Die Isolierung des Reaktionsproduktes ist dabei zwangsläufig mit dem Anfall großer Mengen phosphororganischer Nebenprodukte verbunden, die abgetrennt und entsorgt werden müssen, da ein Recyclingprozess mit wirtschaftlich vertretbarem Aufwand nicht möglich ist.
Die Durchführung chemischer Reaktionen mit toxischen, umweltgefährdenden und ekelerregend riechenden Hilfsstoffen sowie der Anfall größerer Mengen Abprodukte belastet die Verfahren in technischer und wirtschaftlicher Weise außerordentlich, da garantiert werden muß, daß die betreffenden Schadstoffe und Abprodukte nicht entweichen, sondern durch Aufarbeitung wiederverwendbar gemacht oder mit außerordentlichen Mitteln entsorgt werden können. Durch die Verwendung der Verbindungen und Verfahren werden diese Nachteile des bisher bekannten Verfahrens zur Herstellung von Desogestrel überwunden.

FR-A-2209577 offenbart Steroide, welche den vorliegenden strukturell nahkommen. Da diese Verbindengenjedoch für andere Reaktionen verwendet werden, würde sie ein Fachmann für die vorliegende Aufgabe nicht in Betracht ziehen.

Die erfindungsgemäßen Verbindungen der allgemeinen Formel I werden aus 18a-Hydroxy-Steroiden erhalten, die totalsynthetisch im industriellen Maßstab zugänglich sind (US-PS 3 391 170).

Das 11α-Hydroxy-Steroid der Formel II 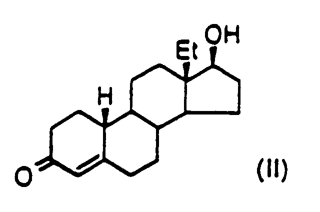 aus welchem die erfindungsgermaßen Verbindungen der allgemeinen Formel Ia bis If hergestellt werden, wird nach der UK-PS 1 128 044 aus totalsynthetisch zugänglichem 3-Methoxy-11a-homo-estra-1,3,5(10),9(11)-tetraen-17β-ol gewonnen.

Durch die Verwendung der erfindungsgemäßen Verbindungen der allgemeinen Formel Ia bis If die bereits eine 13-Ethyl-Gruppe im Ringgerüst tragen, ist ein mehrstufiges Verfahren zur Homologisierung der 13-Methyl-Gruppe unter Einschluß einer Acylhypoiodit-Reaktion, die zu einem 11,17-Lacton führt, nicht erforderlich, um Desogestrel herzustellen. Damit vermeidet man den Einsatz toxischer und umweltgefährdender Hilfsstoffe, Prozesse zum Recycling dieser Stoffe oder deren Entsorgung.

Ein weiterer Vorteil der erfindungsgemäßen Verbindungen der allgemeinen Formel Ia bis If die Tatsache, daß sie eine reduktive Eliminierung der 3-Oxo-Gruppe aus dem Steroidmolekül erlauben, ohne daß hierzu ein 3-Thioacetal gebildet werden muß. Eine reduktive Eliminierung der 3-Oxo-Gruppe aus dem Steroid-Molekül kann somit ohne Verwendung des ekelerregend riechenden Ethandithiols erfolgen.

Von weiterem Vorteil der erfindungsgemäßen Verbindungen ist, daß die reduktive Eliminierung der 3-Oxo-Gruppe. die mit ihrer Hilfe möglich ist, 3-Desoxy-Verbindungen ergibt, die praktisch frei von Doppelbindungsisomeren sind. Es ist bekannt, daß die reduktive Spaltung von Thioacetalen, die sich von 4-En-3-oxo-Steroiden ableiten, zu 4-En-Steroiden führt, die mit 3-En-Steroiden verunreinigt sind.

Wegen der strukturellen Ähnlichkeit der Doppelbindungsisomeren ist die Reindarstellung des gewünschten 4-En-Steroids nur mit hohem Aufwand durchzuführen, z.B. über Dibromide (D.H.R. Barton, W.J. Rosenfelder, J. Chem. Soc. 1951, 1048).

Das erfindungsgemäße Prinzip der Olefinierung einer 11-Oxo-Gruppe ermöglicht schließlich, den Einsatz des Olefinierungsmittels und damit den zwangsläufigen Anfall von phosphororganischen Nebenprodukten stark zu reduzieren, indem der Olefinierungsprozeß unter Einwirkung von Ultraschall durchgeführt wird. Erfindungsgemaß wird das 11α-Hydroxy-Steroid der allgemeinen Formel II in an sich bekannter Weise mit einem komplexen Metallhydrid reduziert, wobei das Trihydroxy-Steroid der Formel Ia erhalten wird.

Das Trihydroxy-Steroid der Formel Ia wird in an sich bekannter Weise mit Methanol in Gegenwart von p-Toluensulfonsäure zur Reaktion gebracht, wobei der Methylether der allgemeinen Formel Ia gebildet wird.

Durch Einwirkung von Säure auf das Trihydroxy-Steroid der Formel Ia entsteht das dien-Steroid der allgemeinen Formel Ib.

Das Dien-Steroid oder der Methylether der allgemeinen Formel Ia wird mit einem Alkalimetall, wie Lithium oder Natrium, oder mit einem Erdalkalimetall, wie Calcium, in flüssigem Ammoniak, in einem niederen Amin oder in Ethylendiamin in das Dihydroxy-Steroid der allgemeinen Formel Ic umgewandelt.

Das Dihydroxy-Steroid der allgemeinen Formel Ic wird in an sich bekannter Weise, zum Beispiel mit einem Chrom-VI-Reagenz oder mit Dimethylsulfoxid in Gegenwart eines Aktivators wie Oxalylchlorid, Pyridin-Schwefeltrioxid-Komplex oder Phosphor-V-oxid, und eines Amins in das Dioxo-Steroid der Formel Id umgewandelt.

Auf das Dioxo-Steroid der Formel Id läßt man Ethan-1,2-diol oder (2,2-Dimethyl)propan-1,3-diol und Ethylorthoformiat in Gegenwart einer Säure einwirken oder man bringt das Dioxo-Steroid der Formel Id mit Ethan-1,2-diol oder (2,2-Dimethyl)propan-1,3-diol in Gegenwart einer Säure und eines Lösungsmittels zur Reaktion, mit dessen Hilfe das Reaktionswasser azeotrop aus dem Reaktionsgemisch entfernt werden kann, wobei die Oxoacetale der Formel Ie gebildet werden.

Erfindungsgemäß behandelt man die Oxo-Acetale der Formel Ie mit 3 Moläquivalent Methylen-triphenylphosphoran unter der Einwirkung von Ultraschall, wobei die Methylen-Acetale der Formel If gebildet werden. Die Dauer der Ultraschalleinwirkung beträgt 8 Stunden bis 10 Stunden bei einer Reaktionstemperatur von 60 - 80 °C. Bei der bisher bekannten WITTIG-Olefinierung von 3-Desoxy-11-oxosteroiden zu 3-Desoxy-11,11-methylensferoiden mit Methylen-triphenylphosphoran betragen die Reaktionszeiten 20 bis 22 Stunden bei einer Reaktionstemperatur von 60 °C, wobei ein Überschuß bis zu 8 Moläquivalent Phosphoran zur Anwendung kommt. (DOS 2 361 120; A. J. v.d. Broek et al.: Recl. Trav. chim. Pays-Bas 94 (1975) 35).

Die Oxoacetale der Formel Ie stellen wertvolle Ausgangsstoffe für die Herstellung von 13-Ethyl-11-methylen-18,19-bisnor-17α-pregn-4-en-20-in-17-ol (Formel IV = Desogestrel) dar, indem die Acetalgruppe hydrolytisch abgespalten wird, wobei die Verbindung der Formel III entsteht, die nach Umsetzung mit Lithiumacetylid Verbindung IV ergibt.

Das erfindungsgemäße Verfahren wird durch das nachfolgende Formelschema verdeutlicht. 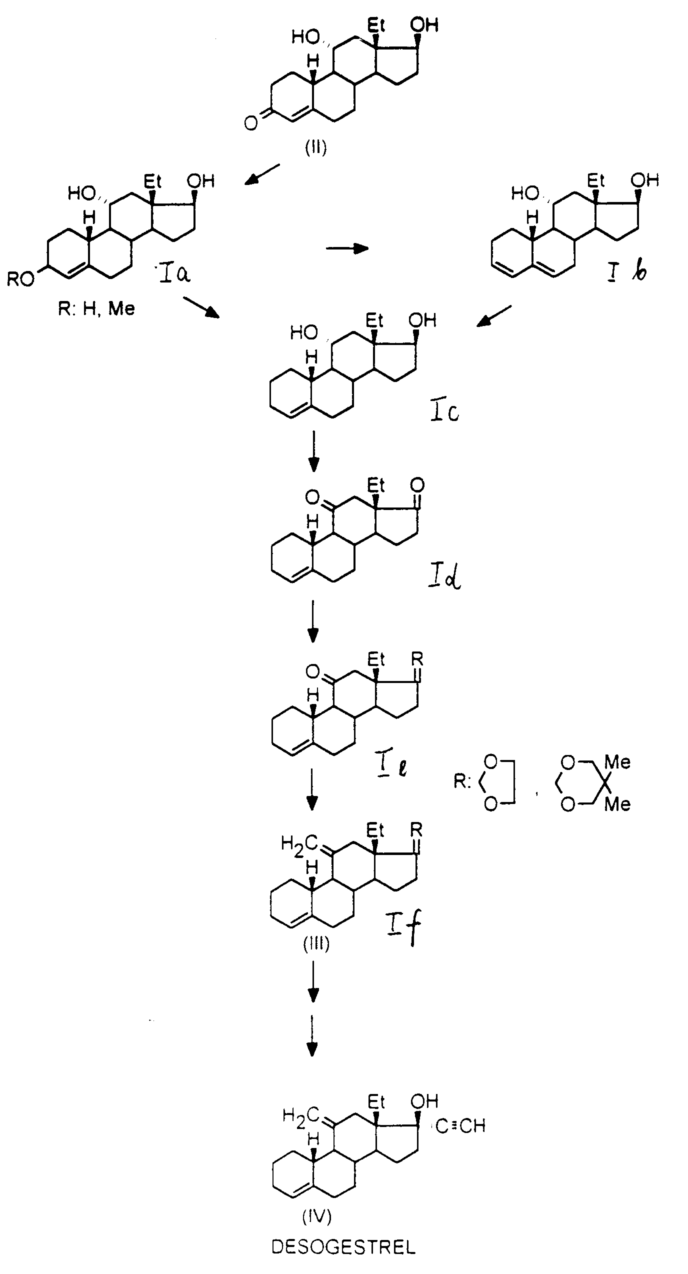 Die erfindungsgemäßen Verbindungen und ihre Herstellung werden nachfolgend beispielhaft beschrieben.

### Beispiel 1

### 18a-Homo-estr-4-en-3,11α,17β-triol

11α,17β-Dihydroxy-18a-homo-estr-4-en-3-on (1 g; 3,3 mmol) und CeCl₃ . 7H₂O (1,26 g; 3,4 mmol) werden in Methanol (10 ml) gelöst. Die Lösung kühlt man auf 0 °C ab, danach wird portionsweise Natriumborhydrid (245 mg; 6,5 mmol) zugegeben. Nach 30 minütiger Reaktionszeit wird mit wäßriger Essigsäure (50 %) neutralisiert und die Lösung im Vakuum weitgehend eingeengt. Den Rückstand verteilt man zwischen Wasser und Ethylacetat. Die organische Phase wird mit gesättigter wäßriger Natriumchloridlösung gewaschen, über wasserfreiem Natriumsulfat getrocknet und im Vakuum zur Trockne eingeengt.
Man erhält 18a-Homo-estr-4-en-3,11α,17β-triol als Schaum, der in die nächste Stufe eingesetzt werden kann.
Durch Chromatographie an Kieselgel mit Ethylacetat als Eluent erhält man 18a-Homo-estr-4-en-3α,11α,17β-triol vom Fp. 180 °C bis 185 °C und 18a-Homo-estr-4-en-3β,11α,17β-triol vom Fp. 133,5 °C bis 138 °C.

### Beispiel 2

### 3-Methoxy-18a-homo-estr-4-en-11α,17β-diol

18a-Homo-estr-4-en-3,11α,17β-triol (1 g; 3,3 mmol) löst man in Methanol. Zu der Lösung fügt man p-Toluolsulfonsäure (10 mg; 0,06 mmol) zu, dann läßt man das Gemisch 1 Stunde bei Raumtemperatur stehen. Danach gibt man wäßrige gesättigte Natriumhydrogencarbonat-Lösung (0,5 ml) zu und destilliert das Methanol im Vakuum ab.
Der Rückstand wird in Ethylacetat aufgenommen, die organische Phase mit wäßriger gesättigter Natriumchloridlösung gewaschen und über wasserfreiem Natriumsulfat getrocknet. Nach Einengen zur Trockne erhält man 3-Methoxy-18a-homo-estr-4-en-11α,17β-diol als Schaum, der in die nächste Stufe eingesetzt werden kann.
Durch Chromatographie an Kieselgel mit Ethylacetat als Eluent erhält man 3α-Methoxy-18a-homo-estr-4-en-11α,17β-diol vom Fp. 150°C bis 154 °C und 3β-Methoxy-18a-homo-estr-4-en11α,17β-diol vom Fp.100 °C bis 106 °C.

### Beispiel 3

### 18a-Homo-estra-3,5-dien-11α,17β-diol

18a-Homo-estr-4-en-3,11α,17β-triol (770 mg; 2,5 mmol) und p-Toluensulfonsäure (140 mg; 0,8 mmol) werden in Methanol (7 ml) gelöst. Die Lösung erwärmt man 2 Stunden auf +50 °C, danach wird auf Raumtemperatur abgekühlt, mit wäßriger gesättigter Natriumhydrogencarbonat-Lösung neutralisiert und im Vakuum weitgehend eingeengt.
Den Rückstand versetzt man mit Wasser, wobei ein kristalliner Niederschlag erhalten wird, der abgesaugt, getrocknet und aus Methanol umkristallisiert wird. Man erhält 18a-Homo-estra-3,5-dien-11α,17β-diol vom Fp. 145 °C bis 148 °C.
Die gleiche Verbindung wird erhalten, wenn man von 3-Methoxy-18a-homo-estr-4-en-11α,17β-diol ausgeht.

### Beispiel 4

### 18a-Homo-estr-4-en-11α,17β-diol

a) Aus 3-Methoxy-18a-homo-estr-4-en-11α,17β-diol
   Zu einem Gemisch von Ethylamin (1032 ml) und Lithium (11,44 g; 1,65 g-Atom) wird eine Lösung von 3-Methoxy-18a-homo-estr-4-en-11α,17β-diol (120 g; 0,37 mol) in Tetrahydrofuran (240 ml) so zugetropft, daß die Reaktionstemperatur +10 °C nicht übersteigt. Anschließend läßt man noch bis zu 1 Stunde nachreagieren, zersetzt dann das Reaktionsgemisch durch vorsichtige Zugabe von Ammoniumchlorid (90 g) und destilliert das Ethylamin unter schonenden Bedingungen weitestgehend ab. Der Rückstand wird in Tetrahydrofuran aufgenommen, die Tetrahydrofuranlösung mehrfach mit Alkalilauge ausgeschüttelt und dann im Vakuum eingeengt. Der Destillationsrückstand wird mit Methyl-tert.-butylether versetzt und zur Kristallisation gebracht. Das Kristallisat wird abgesaugt, mit wenig kaltem Methyl-tert.-butylether gewaschen und dann getrocknet.
   Das erhaltene Kristallisat wird aus Toluol umkristallisiert. Man erhält 18a-Homo-estr-4-en-11α,17β-diol vom Fp. 118,5 °C bis 121 °C.
b) Aus 18a-Homo-estra-3,5-dien-11α,17β-diol
   Zu einem Gemisch von Ammoniak (35 ml) 18a-Homo-estra-3,5-dien-11α,17β-diol (1,4 g; 4,85 mmol), Tetrahydrofuran (25 ml) und Isopropanol (1 ml) wird bei Temperaturen unterhalb -45 °C Natrium (440 mg; 0,019 g-Atom) zugesetzt. Nach einer Gesamtreaktionszeit von 2 Stunden wird das Reaktionsgemisch durch Zusatz von Ammoniumchlorid (1 g) zersetzt, das Ammoniak abdestilliert und der Rückstand unter Zusatz von Wasser (20 ml) und konz. Alkalilauge aufgearbeitet. Nach Abtrennung der wäßrigen Phase engt man die Tetrahydrofuranlösung am Vakuumrotationsverdampfer zur Trockne ein.
   Reinigung über Bis-trimethylsilylether und Umkristallisation aus Toluol ergibt 18a-Homo-estr-4-en-11α,17β-diol vom Fp. 119 °C bis 121 °C.

### Beispiel 5

### 18a-Homo-estr-4-en-3,17-dion

a) 18a-Homo-estr-4-en-11α,17β-diol (1 g; 3,44 mmol) wird in Aceton (6 ml) gelöst uns bei 0 °C mit 8N-Chromschwefelsäure (3 ml) versetzt. Man rührt 3 Stunden bei 0 °C, setzt Wasser (100 ml) zu und destilliert das Aceton im Vakuum ab. Es wird abgesaugt und mit Wasser gewaschen. Man erhält 18a-Homo-estr-4-en-3,17-dion vom Fp. 151 °C bis 153 °C.
b) 18a-Homo-estr-4-en-11α,17β-diol (10 g; 34,4 mmol) wird in Triethylamin (40 ml; 288 mmol) und Dimethylsulfoxid (34,7 ml; 488 mmol) gelöst. Dann gibt man unter Rühren Schwefeltrioxid-Pyridin-Komplex (20 g; 126 mmol) bei Raumtemperatur hinzu. Das Reaktionsgemisch wird 3 weitere Stunden bei Raumtemperatur gerührt und dann mit Wasser (250 ml) verdünnt. Man extrahiert mit Toluen (3 x 50 ml), wäscht die vereinigten Extrakte mit gesättigter wäßriger Natriumchlorid-Lösung, trocknet mit wasserfreiem Natriumsulfat und engt im Vakuum ein. Umkristallisation des Rückstandes aus Methanol ergibt 18a-Homo-estr-4-en-3,17-dion vom Fp. 154 °C bis 155 °C.

### Beispiel 6

### 17,17-Ethylendioxy-18a-homo-estr-4-en-11-on

18a-Homo-estr-4-en-11,17-dion (1 g; 3,49 mmol), Ethylenglykol (1 ml; 17,9 mmol), Triethylorthoformiat (2 ml; 12,0 mmol) und p-Toluolsulfonsäure (0,06 g; 0,315 mmol) werden bei 40 °C miteinander verrührt. Nach 5 Stunden wird mit Toluol (100 ml) verdünnt und die Lösung mit 5%iger wäßriger Natriumhydrogencarbonat-Lösung (6 x 50 ml) gewaschen, es wird über wasserfreiem Natriumsulfat getrocknet und engeengt. Der Rückstand wird aus Essigester/n-Hexan umkristallisiert, und man erhält 17,17-Ethylendioxy-18a-homo-estr-4-en-11-on vom Fp. 98 °C bis 100 °C.

### Beispiel 7

### 17,17-(2,2-Dimethyl)trimethylendioxy-18a-homo-estr-4-en-11-on

18a-Homo-estr-4-en-11,17-dion (1 g; 3,49 mmol), 2,2-Dimethylpropan-1,3-diol (1 g, 9,60 mmol), Triethylorthoformiat (2 ml; 12 mmol) und p-Toluolsulfonsäure (0,06 g; 0,315 mmol) werden bei 40 °C miteinander verrührt.
Nach 6 Stunden wird das ausgefallene Produkt abgesaugt und mit kaltem Ethanol (5 ml; 0 °C) gewaschen. Man erhält 17,17-(2,2-Dimethyl)trimethylendioxy-18a-homo-estr-4-en-11-on vom Fp. 127 °C bis 130 °C.

### Beispiel 8

### 11-Methylen-18a-homo-estr-4-en-17-on

a) Unter Argonschutz und Feuchtigkeitsausschluß werden in ein 6 Liter-Reaktionsgefäß, das in einem Ultraschallbad (25 kHz) angeordnet ist, nacheinander absolutes Toluen (1,2 l), Methyl-triphenylphosphoniumjodid (1,18 kg; 2,92 mol), Natriumhydrid 80%ig (86,5 g; 2,88 mol) und absolutes Dimethylsulfoxid (1,6 l) eingetragen. Man beginnt mit der Beschallung und erwärmt das Gemisch auf +70 °C. Nach beendeter Wasserstoffentwicklung läßt man zu der Ylidlösung eine Lösung von 17,17-Ethylendioxy-18a-homo-estr-4-en-11-on (317,4 g; 0,96 mol) in Toluen (400 ml) zulaufen. Die Beschallung wird 8 bis 10 Stunden lang fortgesetzt, wobei man eine Reaktionstemperatur von +70 °C einhält. Danach destilliert man aus dem Reaktionsgemisch erst das gesamte Toluen und dann Dimethylsulfoxid (ca. 0,5 l) im Vakuum ab. Der Destillationsrückstand wird auf +30 °C abgekühlt und vorsichtig unter Rühren mit Wasser (1,5 l) zersetzt. Die entstandene Lösung extrahiert man mit Cyclohexan (3 x 1 l), die vereinigten Extrakte werden mit Wasser gewaschen (2 x 1 l), auf ein Volumen von 1,5 l eingeengt, über neutrales Aluminiumoxid (500 g) filtriert und im Vakuum eingeengt. Der Rückstand besteht aus 17,17-Ethylendioxy-11-methylen-18a-homo-estr-4-en. Diesen Rückstand löst man in einem 1 : 1 Gemisch von Ethylacetat und Methanol, dem man unter Rühren p-Toluensulfonsäure-Hydrat(10 g) zufügt. Man rührt noch 2 weitere Stunden bei Raumtemperatur, dann wird bis zur Neutralisation gesättigte wäßrige Natriumhydrogencarbonatlösung zugegeben und die organische Phase abgetrennt. Nach dem Einengen im Vakuum kristallisiert man den Rückstand aus Methanol um, wobei 11-Methyen-18a-homo-estr-4-en-17-on vom Fp. 101 °C bis 102,5 °C erhalten wird.
   (DOS 2 361 120: 96 °C bis 99 °C).
b) 17, 17-(2,2-Dimethyl)trimethylendioxy-18a-homo-estr-4-en-11-on ( 36 g; 96 mmol) wird in Analogie zu Beispiel 10 mit dem Methyl-triphenylphosphoniumjodid (118 g; 0,292 mol) und Natriumhydrid 80%ig (8,65 g; 0,288 mol) in Dimethylsulfoxid/Toluen (160 ml/120 ml) olefiniert und das gebildete 11-Methylen-acetal hydrolytisch gespalten, wobei man 11-Methylen-18a-homo-estr-4-en-17-on erhält, das mit der nach Beispiel 8a erhaltenen Verbindung identisch ist.

### Beispiel 9

Acetylen wurde 2 Stunden lang durch eine Lösung von Lithium (10 g; 1,44 g-Atom) in Ethylendiamin (200 ml) eingeleitet. Dann gab man eine Lösung von 11-Methylen-18a-homo-estr-4-en-17-on (10 g; 35 mmol) in Tetrahydrofuren (100 ml) zu, wonach noch 2 Stunden bei einer Reaktionstemperatur von +25 °C und weiterem Einleiten von Acetylen gerührt wurde. Anschließend verdünnt man mit Ethylacetat (250 ml) und neutralisiert unter Kühlen mit Schwefelsäure (20 %).
Aufarbeitung der organischen Phase, Chromatographie des Produkts an Kieselgel (Eluent: Dichlormethan) und Umkristallisation der gereinigten Verbindung aus n-Hexan ergab Desogestrel; Fp. 109 °C bis 111 °C (DOS 2 361 120: 109 °C bis 110 °C).

## Patentansprüche

1. Steroidzwischenprodukte dar Formeln Ia bis If

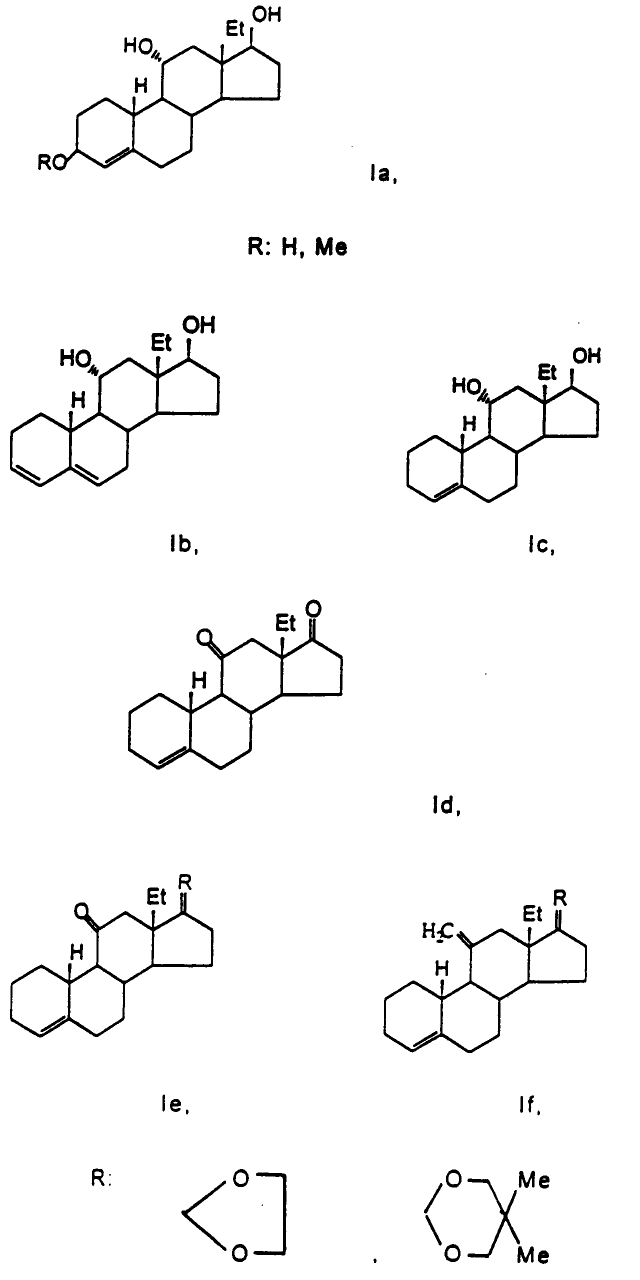

2. Verfahren zur Herstellung der Steroidzwischenprodukte Ia bis If gemäß Anspruch 1,
dadurch gekennzeichnet, daß man
entweder die Verbindung Ia oder die Verbindung Ib mit einem Alkali- oder Erdalkalimetall in flüssigem Ammoniak, in einem niederen Amin oder in Ethylendiamin umsetzt,
die so erhaltene Verbindung Ic mit Chrom-VI-Reagenzien oder mit Dimethylsulfoxid in Gegenwart eines Aktivators, wie Oxalylchlorid, Pyridin-Schwefeltrioxid-Komplex oder Phosphor-V-oxid, und eines Amins zur Reaktion bringt,
die so erhaltene Verbindung Id mit Ethan-1,2-diol oder (2,2-Dimethyl)propan-1,3-diol in Gegenwart einer Säure unter azeotropem Entfernen des sich bildenden Wassers mit Toluol, umsetzt
und schließlich die so erhaltene Verbindung Ie mit Methylentriphenylphosphoran unter der Einwirkung von Ultraschall zur Reaktion bringt.

## Claims

1. Steroid intermediates of the formulae Ia to If

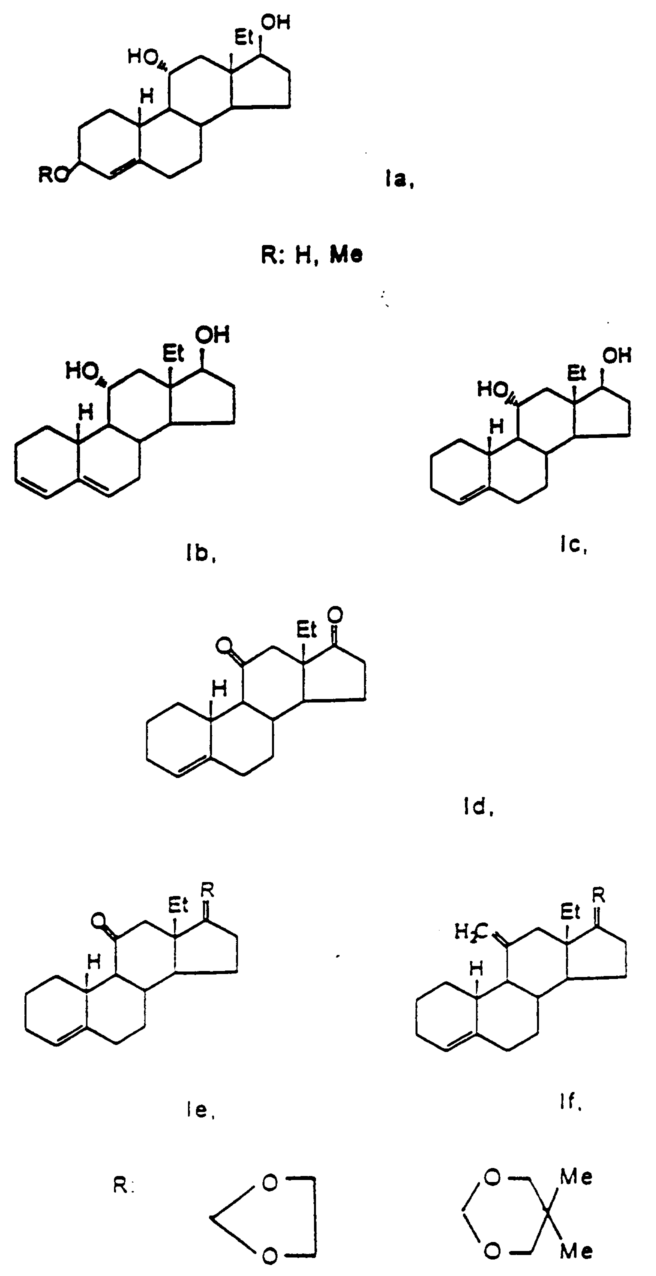

2. A process for producing the steroid intermediates Ia to If according to claim 1,
characterised in that
either the compound Ia or the compound Ib is reacted with an alkali metal or alkaline-earth metal in liquid ammonia, in a lower amine or in ethylenediamine,
the compound Ic thus obtained is caused to react with chromium (VI) reagents or with dimethyl sulfoxide in the presence of an activator, such as oxalyl chloride, pyridine/sulfur trioxide complex or phosphorus(V) oxide and an amine,
the compound Id thus obtained is reacted with 1,2-ethanediol or (2,2-dimethyl)-1,3-propanediol in the presence of an acid with azeotropic removal using toluene of the water formed,
and finally the compound Ie thus obtained is caused to react with methylene triphenyl phosphorane through the action of ultrasound.

## Revendications

1. Produits intermédiaires stéroïdes des formules Ia à If

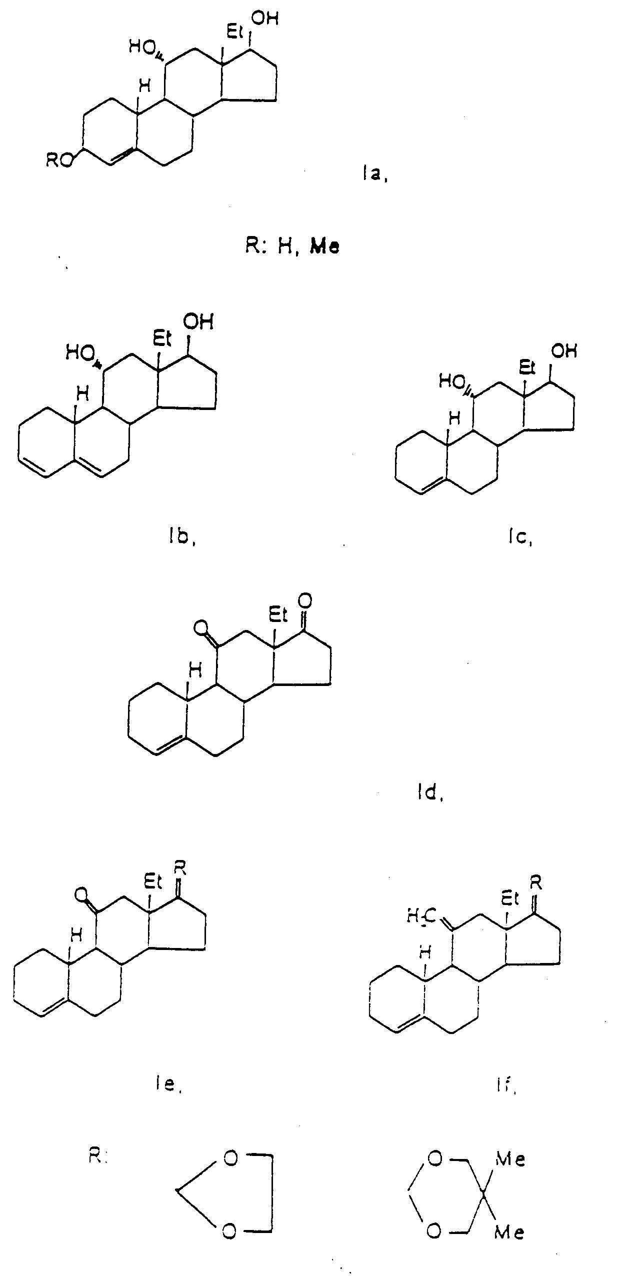

2. Procédé de production des produits intermédiaires stéroïdes Ia à If selon la revendication 1,
caractérisé en ce qu'on transforme le composé Ia ou le composé Ib avec un métal alcalin ou un métal alcalino-terreux dans de l'ammoniac liquide en une amine inférieure ou de l'éthylènediamine,
le composé ainsi obtenu Ic est mis à réagir avec des réactifs de chrome-VI ou du diméthylsulfoxyde en présence d'un activateur tel que du chlorure d'oxalyle, un complexe de pyridine-trioxyde de soufre ou un oxyde de phosphore-V et une amine,
le composé ainsi obtenu Id est transformé avec de l'éthane-1,2-diol ou du (2,2-diméthyl)propane-1,3-diol en présence d'un acide avec élimination azéotropique de l'eau qui se forme avec du toluène et
enfin le composé ainsi obtenu le est mis à réagir avec du méthyléne triphénylphosphorane sous l'effet d'ultrasons.
